(19) Europäisches Patentamt
European Patent Office
Office européen des brevets

(11) Veröffentlichungsnummer: **0 325 967 B1**

(12) **EUROPÄISCHE PATENTSCHRIFT**

(45) Veröffentlichungstag der Patentschrift: **13.04.94**

(51) Int. Cl.[5]: **C07D 317/20**, C07D 317/28, C07D 317/30, C07D 317/46, C25B 3/00, C07C 53/00

(21) Anmeldenummer: **89100588.6**

(22) Anmeldetag: **13.01.89**

(54) **Verfahren zur Herstellung von enantiomereneinen 2,2,4-trisubstituierten 1,3-Dioxolanen.**

(30) Priorität: **26.01.88 CH 253/88**

(43) Veröffentlichungstag der Anmeldung:
**02.08.89 Patentblatt 89/31**

(45) Bekanntmachung des Hinweises auf die Patenterteilung:
**13.04.94 Patentblatt 94/15**

(84) Benannte Vertragsstaaten:
**AT BE CH DE ES FR GB IT LI NL SE**

(56) Entgegenhaltungen:
**EP-A- 0 143 973**
**DE-A- 2 810 732**

**JOURNAL OF THE AMERICAN CHEMICAL SOCIETY, Band 102, Nr. 20, Seiten 6304-6311, Washington, DC, US; M.E. JUNG et al.: "Total synthesis of (R)-glycerol acetonide and the antiepileptic and hypotensive drug (-)-gamma-amino-beta-hydroxybutyric acid (GABOB): Use of vitamin C as a chiral starting material"**

(73) Patentinhaber: **LONZA AG**
**Gampel/Wallis**
**Geschäftsleitung Basel**
**CH-4002 Basel(CH)**

(72) Erfinder: **Voeffray, Robert, Dr.**
**Florastrasse 37**
**Basel(CH)**

(74) Vertreter: **Weinhold, Peter, Dr. et al**
**Patentanwälte Dr. V. Schmied-Kowarzik**
**Dipl.-Ing. G. Dannenberg**
**Dr. P. Weinhold**
**Dr. D. Gudel**
**Dipl.-Ing. S. Schubert**
**Dr. P.**
**Barz**
**Siegfriedstrasse 8**
**D-80803 München (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Enantiomerenreine 2,2,4-trisubstituierte 1,3-Dioxolane der allgemeinen Formel

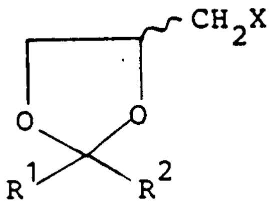

I

mit X = OH oder X = $NHR^3$ sind als Derivate des Glycerins bzw. des 3-Amino-1,2-propandiols äusserst wertvolle chirale Bausteine für stereospezifische Synthesen von Naturstoffen und von anderen optisch aktiven Verbindungen, wie beispielsweise pharmazeutischen Wirkstoffen.

Einige Beispiele dieser Anwendungen sind die Synthesen von (R)-4-Amino-3-hydroxybuttersäure (GABOB) (J.Am.Chem.Soc. 102, 6304 [1980]), von L-Carnitin (EP Anm. 0 060 595), von Acyclovir-Analogen (J.Med.Chem. 28, 926 [1985]) oder von β-Blockern (DE-OS 28 10 732).
Obwohl einige dieser 1,3-Dioxolane, nämlich die Acetonide des Glycerins ($R^1$ = $R^2$ = $CH_3$, X = OH) und einige davon abgeleitete Ether und Ester (X = $OCH_2C_6H_5$, O-Tosyl) bereits kommerziell erhältlich sind, steht der bisher noch sehr hohe Preis dieser Verbindungen einer Anwendung in grösserem Umfang im Wege.
Die zahlreichen, bisher bekannten Herstellungsverfahren gehen von teuren Edukten (L-Serin, L-Arabinose) aus und/oder verwenden teure Reagenzien (Blei(IV)-acetat, $NaIO_4$, Bismut-Verbindungen u.a.) und lassen daher eine Produktion in industriellem Massstab und zu attraktiven Preisen schon aus Kostengründen nicht zu.

Ein neueres Verfahren zur Herstellung von (S)-2,2-Dimethyl-1,3-dioxolan-4-carbaldehyd (L-Glycerinaldehyd-acetonid) (EP Anm. 0 143 973), aus dem durch Reduktion, beispielsweise mit Natriumborhydrid, die entsprechende Hydroxyverbindung erhalten werden kann, geht von 3,4-O-Isopropyliden-L-threonsäure aus, die mit hypochloriger Säure bzw. Hypochlorit in saurem Medium zum L-Glycerinaldehyd-acetonid abgebaut wird.

Obwohl 3,4-O-Isopropyliden-L-threonsäure von L-Ascorbinsäure ausgehend leicht zugänglich und Hypochloritlösung eine billige Chemikalie ist, besitzt dieses Verfahren doch einige schwerwiegende Nachteile:

- Die kommerziell erhältlichen Hypochloritlösungen weisen keinen genau definierten Hypochlorit-Gehalt auf und sind nicht völlig stabil.
- Die bei der Durchführung des Verfahrens gebildete freie hypochlorige Säure ist noch weniger stabil und zersetzt sich teilweise, weshalb mehr als die theoretisch erforderliche Menge verbraucht wird.
- Ein nach Ablauf der Oxidation noch vorhandener Ueberschuss an Hypochlorit muss durch Zugabe eines Reduktionsmittels zerstört werden. Dadurch werden weitere Fremdsubstanzen in das Reaktionsgemisch eingebracht.
- Die Hypochloritlösung ist sehr korrosiv und erfordert für die verwendete Apparatur ein entsprechend beständiges Material.
- Bereits die eingesetzte Hypochloritlösung enthält grosse Mengen Chlorid, weiteres Chlorid entsteht bei der Reaktion. Diese gesamte Chloridmenge muss schliesslich entsorgt werden, ausserdem wird möglicherweise durch die hohe Salzkonzentration die Aufarbeitung des Reaktionsgemisches gestört.
- Die Durchführung der Oxidation in saurer Lösung kann bei empfindlichen Ketalen und insbesondere Acetalen zur Hydrolyse der Ketal- bzw. Acetal-Funktion führen und das Verfahren in diesen Fällen unbrauchbar machen.

Es ist daher wünschenswert, ein Verfahren zu besitzen, das die genannten Nachteile nicht aufweist und für die Herstellung einer Vielzahl von verschiedenartig substituierten enantiomerenreinen 2,2,4-trisubstituierten 1,3-Dioxolanen der allgemeinen Formel I angewandt werden kann. Erfindungsgemäss wird diese Aufgabe gemäss Patentanspruch 1 gelöst.

Die oxidative Decarboxylierung einer Threonsäure oder Erythronsäure der allgemeinen Formel

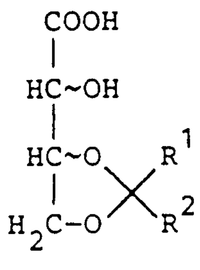

II

durch Elektrolyse liefert unter Erhalt der Konfiguration am β-Kohlenstoffatom in guter Ausbeute das entsprechende Glycerinaldehyd-Derivat. Ein wesentlicher Vorteil dieses elektrochemischen Verfahrens liegt darin, dass neben dem gewünschten Produkt nur Kohlendioxid und Wasserstoff entstehen, die als Gase von selbst aus dem Elektrolyt entweichen. Auch kann kein Ueberschuss eines Oxidationsmittels auftreten, der nach Beendigung der Reaktion beseitigt werden müsste.

Als Reste $R^1$ und $R^2$ können Wasserstoff oder niedrige Alkylgruppen, vorzugsweise Methyl oder Ethyl, insbesondere Methyl, oder Arylgruppen, insbesondere Phenyl, oder Arylalkylgruppen, vorzugsweise solche mit einer $C_{1-4}$-Alkylgruppe, insbesondere Benzyl, vorliegen. Weiterhin können $R^1$ und $R^2$ zusammen eine 1,4-Butandiyl- oder insbesondere 1,5-Pentandiyl-Gruppe bilden, so dass sich zusammen min dem Kohlenstoffatom der Ketalfunktion ein fünf- oder sechsgliedriger Ring ergibt.

Je nach der Anzahl der möglichen Kohlenstoffatome sind die Alkylsubstituenten $R_1$, $R_2$ und $R_3$ , also gerad- oder verzweigtkettige Methyl-, Ethyl-, Propyl-, Butyl-, Pentyl-, Hexyl-, Heptyl- oder Octylgruppen.

Die Elektrolyse erfolgt zweckmässig in einem wässrigen Medium, wie zum Beispiel einem Gemisch von Wasser mit niedrigen Alkoholen, Acetonitril, Dimethylformamid, Dimethylsulfoxid, Hexamethylphosphorsäuretriamid oder Tetrahydrofuran, vorzugsweise jedoch in Wasser ohne zusätzliches Lösungsmittel.

Als Elektrodenmaterial kann für beide Elektroden ein Edelmetall, wie z.B. Platin, Gold oder Iridium, oder Graphit, für die Anode jedoch vorzugsweise Graphit, verwendet werden.

Als Zelle wird zweckmässig eine ungeteilte Zelle benutzt, in der die Lösung kontinuierlich zirkuliert wird. Im Labormassstab ist die Durchführung der Elektrolyse beispielsweise auch an Platinelektroden in einem Becherglas möglich, wobei die Lösung gerührt oder durch eine rotierende Elektrode durchmischt wird. Die Stromdichte beträgt zweckmässig 0,1 bis 320 mA/cm$^2$, vorzugsweise 2 bis 80 mA/cm$^2$; die Elektrolyse wird vorzugsweise bei konstantem Strom durchgeführt.

Bis zum vollständigen Umsatz werden normalerweise 2 bis 2,8 Faraday/mol gebraucht.

Die Temperatur bei der Elektrolyse beträgt zweckmässig 5 bis 90 °C, vorzugsweise 10 bis 70 °C.

Der pH-Wert der Lösung liegt während der Elektrolyse zweckmässig zwischen 4 und 10 und wird durch Zugabe von Säure, vorzugsweise Schwefelsäure, in diesem Bereich gehalten. Vorzugsweise wird die Elektrolyse bei pH 6 bis 7 durchgeführt.

Die Konzentration des Threonsäure- oder Erythronsäure-Derivats liegt bei der Elektrolyse zweckmässig zwischen 0,5 und 25%, sie beträgt vorzugsweise 5 bis 15%.

Als Ausgangsmaterial werden vorzugsweise L-Threonsäure- oder D-Erythronsäure-Derivate eingesetzt, die zweckmässig aus den entsprechenden L-Ascorbinsäure- bzw. D-Isoascorbinsäure-(D-Erythorbinsäure-) -Derivaten der allgemeinen Formel

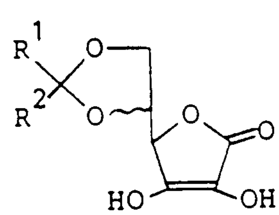

IV

in der $R^1$ und $R^2$ die oben genannten Bedeutungen haben, durch oxidative Spaltung, vorzugsweise mit wässrigem Wasserstoffperoxid in Gegenwart von Calciumcarbonat (EP Anm. 0 111 326), hergestellt werden.

Die L-Threonsäure- bzw. D-Erythronsäure-Derivate fallen bei dem letztgenannten Herstellungsverfahren als wässrige Lösungen ihrer Calciumsalze an, daneben entsteht schwerlösliches Calciumoxalat. Bei der

Elektrolyse der Calciumsalze tritt als störende Nebenreaktion eine Abscheidung von Calciumcarbonat bzw. Calciumhydroxid an den Elektroden ein. Die Calciumsalze werden daher zweckmässig durch Behandlung mit einem Kationenaustauscher in ein Alkalisalz oder in die freien Säuren übergeführt. Die freie Säure kann dann mit einer geeigneten Base, vorzugsweise einem Alkalihydroxid oder einem tertiären Amin, wie z.B. Triethylamin, in ein für die Elektrolyse geeignetes Salz übergeführt werden. Die Umwandlung der Calciumsalze in die freien Säuren kann auch dadurch erfolgen, dass das Calcium in Form eines schwerlöslichen Salzes ausgefällt wird. Dies kann beispielsweise durch Zugabe von Schwefelsäure, die mit Calcium schwerlösliches Calciumsulfat bildet, erreicht werden.

Als besonders günstig hat es sich jedoch erwiesen, bereits die Oxidation der L-Ascorbinsäure- bzw. D-Isoascorbinsäure-Derivate in Gegenwart eines Alkalicarbonats, vorzugsweise Natriumcarbonat, durchzuführen. Dadurch wird die entstehende L-Threonsäure bzw. D-Erythronsäure von vornherein in Form ihres Alkalisalzes erhalten, während das Calcium des ebenfalls zugesetzten Calciumcarbonats lediglich die entstehende Oxalsäure in Form des schwerlöslichen Calciumoxalats bindet. Das Alkalicarbonat wird zweckmässig in einer Menge von 0,5 bis 2 mol (bezogen auf 1 mol des L-Ascorbinsäure- bzw. D-Isoascorbinsäure-Derivats) zugesetzt.

Das durch die Elektrolyse erhaltene Glycerinaldehyd-Derivat kann durch übliche Extraktionsverfahren isoliert und durch Destillation im Vakuum gereinigt werden. Da solche Glycerinaldehyd-Derivate jedoch nur wenig haltbar sind, ist es zweckmässig, sie nicht zu isolieren, sondern direkt im Reaktionsgemisch weiter umzusetzen. Im erfindungsgemässen Verfahren erfolgt diese Umsetzung durch Reduktion der Aldehydfunktion zur Hydroxymethylgruppe oder durch Reduktion in Gegenwart eines primären Amins, vorzugsweise eines niederen Alkylamins, wie z.B. Isopropylamin, unter Bildung einer sekundären Aminogruppe.
Die Reduktion wird vorzugsweise durch katalytische Hydrierung, insbesondere an Raney-Nickel oder Palladium-Katalysatoren, vorgenommen; die Reduktion zur Hydroxymethylgruppe kann ausserdem insbesondere auch mit Natriumboranat erfolgen. Es ist auch möglich, das Glycerinaldehyd-Derivat durch eine andere Reaktion, beispielsweise die Bildung einer Schiff'schen Base mit einem Amin, in eine stabilere Verbindung überzuführen (EP Anm. 0 120 289, EP Anm. 0 143 973).

Beispiel 1

5,6-O-Isopropyliden-L-ascorbinsäure

Zu einer Lösung von 1 ml Acetylchlorid in 40 ml Aceton wurden 10,0 g (55 mmol) L-Ascorbinsäure zugegeben. Das heterogene Gemisch wurde 3 Stunden bei Raumtemperatur und anschliessend 8 Stunden bei 0 bis 5 °C gerührt. Das Produkt wurde abfiltriert, zweimal mit je 5 ml kaltem Aceton gewaschen und getrocknet.
Ausbeute: 9,65 g (81,1%)
Schmelzpunkt: 223 bis 226 °C
$[\alpha]_D^{20}$ = +10,5 ° (c = 5, Methanol)
$^1$H-NMR: ($CD_3OD$, 300 MHz) δ
1,34 (s, 3H); 1,37 (s, 3H); 4,04 (dd, J = 8,5/6,5 Hz, 1H, H-C(6)); 4,17 (dd, J = 8,5/7 Hz, 1H, H-C(6)); 4,33 (ddd, J = 7/6,5/3 Hz, 1H, H-C(5)); 4,67 (d, J = 3 Hz, 1H, H-C(4)).

Beispiel 2

5,6-O-Cyclohexyliden-D-isoascorbinsäure

In einem 200 ml-Dreihalskolben wurden 4,45 g (45,2 mmol) Cyclohexanon, 7,05 g (47,5 mmol) Orthoameisensäuretriethylester, 0,04 g p-Toluolsulfonsäure (Monohydrat), 4,15 g (90 mmol) Ethanol und 95 ml Essigsäureethylester auf 100 °C erwärmt und 1 Stunde auf dieser Temperatur gehalten (Rückfluss). Dabei bildete sich 1,1-Diethoxycyclohexan.
Danach wurden 4,0 g (22,7 mmol) D-Isoascorbinsäure zugegeben und das zunächst heterogene Gemisch 5 Stunden am Rückfluss gekocht, wobei sich eine klare Lösung bildete. Anschliessend wurden 2 g Aluminiumoxid zugegeben, die Suspension noch 1 Stunde bei Raumtemperatur gerührt und über Celite® filtriert, das mit dreimal 5 ml Essigsäureethylester nachgewaschen wurde. Das Filtrat wurde bei 35 °C/30 mbar bis auf 20,5 g eingeengt, wobei sich ein dickflüssiger Brei bildete. Das Produkt wurde durch Zugabe von 20 ml Hexan vollends ausgefällt, abfiltriert und getrocknet.
Ausbeute: 5,05 g (86,8%)
Schmelzpunkt: 177 bis 178,5 °C

$^1H$-NMR: (Aceton-$d_6$, 300 MHz) δ
1,30-1,50 (m, 2H); 1,50-1,70 (m, 8H); 2,97 (m, 2H, OH); 3,77 (dd, J = 8,5/6 Hz, 1H, H-C(6)); 4,01 (dd, J = 8,5/7 Hz, 1H, H-C(6)); 4,42 (ddd, J = 7/6/4 Hz, 1H, H-C(5)); 4,84 (d, J = 4 Hz, 1H, H-C(4)).

Beispiele 3 - 8

Analog zu Beispiel 2 wurden folgende Ketale hergestellt:

Beispiel 3

5,6-O-Cyclopentyliden-D-isoascorbinsäure

Ausbeute: 61%
$^1H$-NMR: ($CD_3OD$, 300 MHz) δ
1,60-2,05 (m, 8H); 3,73 (dd, J = 8,5/6 Hz, 1H, H-C(6)); 3,91 (dd, J = 8,5/7,5 Hz, 1H, H-C(6)); 4,36 (ddd, J = 7,5/6/3 Hz, 1H, H-C(5)); 4,82 (d, J = 3 Hz, 1H, H-C(4)).

Beispiel 4

5,6-O-(1-Ethylpropyliden)-D-isoascorbinsäure

Ausbeute: 57%
$^1H$-NMR: ($CD_3OD$, 300 MHz) δ
0,89 (t, 3H); 0,91 (t, 3H); 1,61 (q, 2H); 1,69 (q, 2H); 3,75 (t, J = 7 Hz, 1H, H-C(6)); 3,99 (t, J = 7Hz, 1H, H-C(6)); 4,45 (td, J = 7/3 Hz, 1H, H-C(5)); 4,88 (d, J = 3 Hz, 1H, H-C(4)).

Beispiel 5

5,6-O-Isopropyliden-D-isoascorbinsäure

Ausbeute: 41%
$^1H$-NMR: ($CD_3OD$, 300 MHz) δ
1,35 (s, 3H); 1,42 (s, 3H); 3,78 (dd, J = 8,5/6,5 Hz, 1H, H-C(6)); 4,00 (dd, J = 8,5/7 Hz, 1H, H-C(6)); 4,44 (ddd, J = 7/6,5/4 Hz, 1H, H-C(5)); 4,83 (d, J = 4 Hz, 1H, H-C(4)).

Beispiel 6

5,6-O-Cyclohexyliden-L-ascorbinsäure

Ausbeute: 67%
$^1H$-NMR: ($CD_3OD$, 300 MHz) δ
1,30-1,48 (m, 2H); 1,48-1,65 (m, 8H); 4,02 (dd, J = 8/6,5 Hz, 1H, H-C(6)); 4,16 (dd, J = 8/7 Hz, 1H, H-C(6)); 4,31 (ddd, J = 7/6,5/3 Hz, 1H, H-C(5)); 4,65 (d, J = 3 Hz, 1H, H-C(4)).

Beispiel 7

5,6-O-Cyclopentyliden-L-ascorbinsäure

Ausbeute: 73%
$^1H$-NMR: ($CD_3OD$, 300 MHz) δ
1,55-1,85 (m, 8H); 4,00 (dd, J = 8,5/6 Hz, 1H, H-C(6)); 4,10 (dd, J = 8,5/7 Hz, 1H, H-C(6)); 4,26 (ddd, J = 7/6,5/3 Hz, 1H, H-C(5)); 4,66 (d, J = 3 Hz, 1H, H-C(4)).

Beispiel 8

5,6-O-(1-Ethylpropyliden)-L-ascorbinsäure

$^1$H-NMR: ($CD_3OD$, 300 MHz) $\delta$
0,80-0,95 (m, 6H); 1,55-1,70 (m, 4H); 4,00 (t, J = 7 Hz, 1H, H-C(6)); 4,18 (t, J = 7 Hz, 1H, H-C(6)); 4,32 (td, J = 7/3 Hz, 1H, H-C(5)); 4,69 (d, J = 3 Hz, 1H, H-C(4)).

Beispiel 9

Calcium-3,4-O-isopropyliden-L-threonat

In einem 200 ml-Dreihalskolben wurden 8,0 g (80 mmol) Calciumcarbonat bei Raumtemperatur in 100 ml Wasser suspendiert und 8,65 g (40 mmol) 5,6-O-Isopropyliden-L-ascorbinsäure innerhalb von 30 Minuten portionsweise zugegeben. Nach Abkühlen auf 5°C wurden 18,15 g Wasserstoffperoxid (30%ige Lösung in Wasser, 160 mmol) innerhalb von 2 Stunden zugetropft, wobei die Temperatur unter 20°C gehalten wurde. Das heterogene Gemisch wurde noch 2 Stunden bei Raumtemperatur und 30 Minuten bei 40°C gerührt und nach Zugabe von 2 g Aktivkohle 1 Stunde auf 85°C erwärmt. Das entstandene Calciumoxalat und die Aktivkohle wurden über Celite® filtriert und das Filtrat bis auf 50 g eingeengt. Durch Zugabe von 60 ml Aceton wurde das Produkt ausgefällt.
Ausbeute: 6,2 g (77,4%) Calcium-3,4-O-isopropyliden-L-threonat • 1/4 $H_2O$.
Schmelzpunkt: >250°C
$[\alpha]_D^{20}$ = +21,5° (c = 1, $H_2O$)

Beispiel 10

Calcium-3,4-O-cyclohexyliden-D-erythronat

Zu einer Suspension von 8,0 g (80 mmol) Calciumcarbonat und 10,2 g (40 mmol) 5,6-O-Cyclohexyliden-D-isoascorbinsäure in 150 ml Wasser wurden innerhalb von 2 Stunden 18,15 g Wasserstoffperoxid (30%ige Lösung in Wasser, 160 mmol) zugetropft, wobei die Temperatur unter 20°C gehalten wurde. Das heterogene Gemisch wurde noch 2 Stunden bei Raumtemperatur und 45 Minuten bei 40°C gerührt und nach Zugabe von 2 g Aktivkohle 1 Stunde auf 90°C erwärmt. Das entstandene Calciumoxalat und die Aktivkohle wurden über Celite® abfiltriert und das Filtrat auf 52,5 g eingeengt. Das Produkt wurde durch Zutropfen von 60 ml Ethanol ausgefällt.
Ausbeute: 8,4 g (86%) Calcium-3,4-O-cyclohexyliden-D-erythronat • 1/2 $H_2O$
Schmelzpunkt: >250°C
$[\alpha]_D^{20}$ = +18,8° (c = 1, Ethanol)

Beispiel 11

(R)-2,2-Dimethyl-1,3-dioxolan-4-methanol aus Calcium-3,4-O-isopropyliden-L-threonat (Elektrolyse in Gegenwart von Triethylamin)

Zu einer Lösung von 23,8 g Calcium-3,4-O-isopropyliden-L-threonat (82%ig, 100 mmol) in 220 ml Wasser wurden 10,0 g Schwefelsäure (50%ig, 50 mmol) zugetropft. Danach betrug der pH 1,95 und die heterogene Mischung wurde noch 10 Minuten bei 0-5°C gerührt. Das ausgefallene Calciumsulfat-Dihydrat wurde abfiltriert und zweimal mit je 10 ml Wasser gewaschen. Das Filtrat wurde mit 6,2 g (60 mmol) Triethylamin versetzt und in einer ungeteilten Elektrolysezelle von 250 ml Volumen an Graphitelektroden bei 15 bis 20°C und einem konstanten Strom von 1,0 A 6,5 Stunden elektrolysiert. Der pH-Wert, der am Ende der Elektrolyse 8,0 bis 8,5 betrug, wurde durch Zugabe von 15 g Dinatriumhydrogenphosphat (Dodecahydrat) auf 7 bis 8 gebracht. Das Gemisch wurde auf 0°C gekühlt, innerhalb von 1,5 Stunden portionsweise mit 7,8 g (200 mmol) Natriumborhydrid versetzt, noch 7 Stunden bei 20°C gerührt und anschliessend filtriert. Das Filtrat wurde sechsmal mit jeweils 200 ml Ether extrahiert, die organischen Phasen über Natriumsulfat getrocknet und eingeengt. Der Rückstand wurde bei 24 mbar über Kaliumhydroxid destilliert.
Ausbeute: 6,95 g (52,6%)
Siedepunkt: 85 bis 87°C/24 mbar
$[\alpha]_D^{20}$ = -15,3° (ohne Lösungsmittel)

Gehalt 98,8% (GC).

Beispiel 12

(R)-2,2-Dimethyl-1,3-dioxolan-4-methanol aus 5,6-O-Isopropyliden-L-ascorbinsäure (Oxidation und Elektrolyse in Gegenwart von Natriumcarbonat)

Zu einer Lösung von 21,6 g 5,6-O-Isopropyliden-L-ascorbinsäure (95%ig, 95 mmol) in 250 ml Wasser wurde innerhalb von 15 Minuten ein Gemisch von 10,0 g (100 mmol) Calciumcarbonat und 15,9 g (150 mmol) Natriumcarbonat portionsweise zugegeben. Das heterogene Gemisch wurde noch 1 Stunde bei Raumtemperatur gerührt und dann auf 10 °C abgekühlt. Hierauf wurden innerhalb von 1 Stunde 44,5 g Wasserstoffperoxid (30%ige Lösung in Wasser, 400 mmol) zugetropft, wobei die Temperatur unter 20 °C gehalten wurde. Das Gemisch wurde noch 1 Stunde bei Raumtemperatur und 1 Stunde bei 40 °C gerührt, anschliessend portionsweise mit 4 g Aktivkohle versetzt und noch 1 Stunde bei 75 °C gerührt. Nach Abkühlen auf 50 °C wurde das Gemisch über Celite® filtriert, das Filtrat in einem thermostatisierten Becher auf 15 °C gekühlt und durch Zugabe von Schwefelsäure auf pH 6,5 eingestellt und während der Elektrolyse auf diesem Wert gehalten. Die Elektrolyse fand in einem Rohr (d = 3 cm) mit 2 Kathoden und 2 Anoden aus Graphit (d = 0,5 cm, l = 24 cm) bei einem Strom von 2,2 A statt, wobei die Lösung mittels einer Pumpe kontinuierlich umgewälzt wurde. Insgesamt wurden 2,5 Faraday/mol umgesetzt. Nach der Elektrolyse wurde der pH-Wert durch Zugabe von 15 g Dinatriumhydrogenphosphat (Dodecahydrat) auf 7 bis 8 eingestellt, das Gemisch auf 0 °C gekühlt und innerhalb von 1,5 Stunden portionsweise mit 7,8 g (200 mmol) Natriumborhydrid versetzt. Anschliessend wurde das heterogene Gemisch noch 4 Stunden bei 20 °C gerührt und dann filtriert. Das Filtrat wurde sechsmal mit je 100 ml Essigsäureethylester extrahiert und das durch Einengen des Extrakts gewonnene Rohprodukt im Vakuum destilliert.
Ausbeute: 7,5 g (56,4%)
$[\alpha]_D^{20}$ = -15,1 ° (ohne Lösungsmittel)
Gehalt: 99,3% (GC).

Beispiel 13

(R)-2,2-Dimethyl-1,3-dioxolan-4-methanol aus 5,6-O-Isopropyliden-L-ascorbinsäure (Elektrolyse an Pt-Elektroden)

Wie in Beispiel 12 beschrieben wurden 9,1 g 5,6-O-Isopropyliden-L-ascorbinsäure (95%ig, 40 mmol) mit Wasserstoffperoxid in Gegenwart von Calciumcarbonat und Natriumcarbonat oxidiert. Die Elektrolyse der so erhaltenen Lösung von Natrium-3,4-O-isopropyliden-L-threonat wurde bei 15 °C in einem thermostatisierten Becher durchgeführt. Als Anode wurde eine rotierende (2000 1/Minute) Platinscheibe (A = 3 $cm^2$) benutzt, die gleichzeitig die Durchmischung der Lösung bewirkte. Als Kathode diente ein Platindrahtnetz (A = 6,2 $cm^2$). Es wurde ein konstanter Strom von 0,6 A bis zu einer Elektrizitätsmenge von 2,5 Faraday/mol durch die Lösung geleitet. Die weiteren Schritte erfolgten wie in Beispiel 12 beschrieben.
Ausbeute: 2,0 g (37,4%)
Siedepunkt: 75 bis 77 °C/12 Torr
$[\alpha]_D^{20}$ = -15,3 ° (ohne Lösungsmittel)
Gehalt: 99,1% (GC).

Beispiel 14

(S)-1,2-O-Cyclohexylidenglycerin aus Calcium-3,4-O-cyclohexyliden-D-erythronat

Eine Lösung von 24,8 g Calcium-3,4-O-cyclohexyliden-D-erythronat (95%ig, 100 mmol; hergestellt nach Beispiel 10) in 160 ml Methanol wurde auf eine Säule von 500 g Dowex® 50W (protonierte Form) gegeben und mit 1,5 l Methanol eluiert. Das Eluat wurde auf 120 g eingeengt und mit einer Lösung von 6,2 g (60 mmol) Triethylamin in 100 ml Wasser versetzt. Die weiteren Schritte wurden wie in Beispiel 11 beschrieben durchgeführt.
Ausbeute: 6,0 g (35%)
Siedepunkt: 87 bis 89 °C/1 Torr, 137 °C/17 Torr
$[\alpha]_D^{20}$ = +7,3 ° (c = 2, Methanol)
$^1$H-NMR: ($CDCl_3$, 300 MHz) δ

1,30-1,50 (m, 2H); 1,50-1,70 (m, 8H); 3,04 (t, J = 6 Hz, 1H, OH); 3,59 (ddd, J = 11,5/6/5,5 Hz, 1H, H-C(3)); 3,70 (ddd, J = 10/6/4,5 Hz, 1H, H-C(3)); 3,77 (dd, J = 8/6,5 Hz, 1H, H-C(1)); 4,03 (dd, J = 8/6,5 Hz, 1H, H-C(1)); 4,23 (tdd, J = 6,5/5,5/4 Hz, 1H, H-C(2)).
Gehalt: 96,6% (GC)

Beispiel 15

(S)-1,2-O-Cyclohexylidenglycerin aus 5,6-O-Cyclohexyliden-D-isoascorbinsäure

Eine Lösung von 25,7 g 5,6-O-Cyclohexyliden-D-isoascorbinsäure (95%ig, 95 mmol; hergestellt nach Beispiel 2) in 250 ml Wasser wurde in Gegenwart von Calciumcarbonat und Natriumcarbonat wie in Beispiel 12 beschrieben mit Wasserstoffperoxid oxidiert und anschliessend elektrolysiert.
Ausbeute: 5,7 g (34,7%)
$[\alpha]_D^{20}$ = +7,4° (c = 5, Methanol)
Gehalt: 98,7% (GC).

Beispiel 16

(S)-1,2-O-Cyclopentylidenglycerin aus 5,6-O-Cyclopentyliden-D-isoascorbinsäure

Zu einer Lösung von 10,2 g 5,6-O-Cyclopentyliden-D-isoascorbinsäure (95%ig, 40 mmol; hergestellt nach Beispiel 3) in 150 ml Wasser wurde innerhalb von 10 Minuten ein Gemisch von 4,0 g (40 mmol) Calciumcarbonat und 6,4 g (60 mmol) Natriumcarbonat portionsweise zugegeben. Anschliessend wurde das heterogene Gemisch noch 1 Stunde bei Raumtemperatur gerührt und dann auf 10°C abgekühlt. Hierauf wurden innerhalb von 1 Stunde 18,15 g Wasserstoffperoxid (30%ige Lösung in Wasser, 160 mmol) so zugetropft, dass die Temperatur unter 20°C blieb. Die Mischung wurde noch 1 Stunde bei Raumtemperatur und 1 Stunde bei 40°C gerührt und dann innerhalb von 30 Minuten mit 2 g Aktivkohle versetzt. Schliesslich wurde noch 1 Stunde bei 85°C gerührt und nach Abkühlen auf 50°C über Celite® filtriert. Das trat wurde in einem thermostatisierten Becher auf 15°C gekühlt und durch Zugabe von Schwefelsäure auf einen pH-Wert von 6,5 gebracht und während der Elektrolyse bei diesem Wert gehalten. Die Elektrolyse wurde in einem Rohr (d = 3 cm) an 2 Kathoden und 2 Anoden aus Graphit (d = 0,5 cm, l = 7 cm) mit 1,6 A und insgesamt 2,5 Faraday/mol durchgeführt, wobei die Lösung mit einer Pumpe kontinuierlich zirkuliert wurde. Anschliessend wurde der pH-Wert durch Zugabe von 6 g Dinatriumhydrogenphosphat (Dodecahydrat) auf 7 bis 8 gebracht und das Gemisch auf 0°C gekühlt. Innerhalb von 1 Stunde wurde 3,1 g (80 mmol) Natriumborhydrid portionsweise zugegeben. Das heterogene Gemisch wurde noch 4 Stunden bei 25°C gerührt und dann filtriert. Das Filtrat wurde fünfmal mit je 50 ml Essigsäureethylester extrahiert und der Extrakt nach Abdestillieren des Lösungsmittels im Vakuum destilliert.
Ausbeute: 2,7 g (42,3%)
Siedepunkt: 86 bis 87°C/1 Torr
$[\alpha]_D^{20}$ = +8,9° (c = 5, Methanol)
$^1$H-NMR: ($CDCl_3$, 300 MHz) δ
1,60-2,00 (m, 8H); 3,55-3,70 (m, 3H); 3,78 (dd, J = 8/7 Hz, 1H, H-C(1)); 4,02 (dd, J = 8/6 Hz, 1H, H-C(1)); 4,20 (dddd, J = 7/6/5/4,5 Hz, 1H, H-C(2)).
Gehalt: 99,1% (GC)

Beispiel 17

(S)-2,2-Diethyl-1,3-dioxolan-4-methanol aus 5,6-O-(1-Ethylpropyliden)-D-isoascorbinsäure

Zu einer Suspension von 4,0 g (40 mmol) Calciumcarbonat, 6,4 g (60 mmol) Natriumcarbonat und 10,3 g 5,6-O-(1-Ethylpropyliden)-D-isoascorbinsäure (95%ig, 40 mmol; hergestellt nach Beispiel 4) in 150 ml Wasser wurden innerhalb von 2 Stunden 18,15 g Wasserstoffperoxid (30%ige Lösung in Wasser, 160 mmol) getropft, wobei die Temperatur durch Kühlung unter 20°C gehalten wurde. Das heterogene Gemisch wurde noch 2 Stunden bei Raumtemperatur und 45 Minuten bei 40°C gerührt und nach Zugabe von 2 g Aktivkohle 1 Stunde auf 85°C erwärmt. Das gebildete Calciumoxalat und die Aktivkohle wurden abfiltriert, das Filtrat in einen auf 40°C thermostatisierten Becher gegeben und mit Schwefelsäure auf pH 6,5 eingestellt und während der Elektrolyse auf diesem Wert gehalten. Die Elektrolyse wurde in einem Rohr (d = 3 cm) an 2 Kathoden und 2 Anoden aus Graphit (d = 0,5 cm, l = 7 cm) bei 1,2 A und insgesamt 2,5

Faraday/mol durchgeführt, wobei die Lösung mit einer Pumpe kontinuierlich umgewälzt wurde. Nach Beendigung der Elektrolyse wurden 6 g Dinatriumhydrogenphosphat (Dodecahydrat) zugesetzt, um den pH-Wert auf 7 bis 8 zu bringen. Das Gemisch wurde auf 0 ° C abgekühlt und innerhalb von 2 Stunden portionsweise mit 3,1 g (80 mmol) Natriumborhydrid versetzt. Nach dieser Zugabe wurde noch 4 Stunden bei 25 ° C gerührt und dann filtriert. Das Filtrat wurde fünfmal mit je 50 ml Essigsäureethylester extrahiert und das nach Abdestillieren des Lösungsmittels aus dem Extrakt erhaltene Rohprodukt im Vakuum destilliert.
Ausbeute: 2,45 g (38,3%)
Siedepunkt: 58 bis 61 ° C/0,2 Torr
$[\alpha]_D^{20}$ = +13,6 ° (c= 5, Methanol)
$^1$H-NMR: ($CDCl_3$, 300 MHz) δ
0,93 (t, 3H); 0,95 (t, 3H); 1,65 (q, 2H); 1,69 (q, 2H); 2,17 (s, 1H, OH); 3,61 (dd, J = 11/5 Hz, 1H, H-C(3)); 3,74 (m, 1H, H-C(3)); 3,75 (dd, J = 8/7 Hz, 1H, H-C(1)); 4,03 (dd, J = 8/7 Hz, 1H, H-C(1)); 4,23 (m, 1H, H-C(2)).
Gehalt: 98,7% (GC)

Beispiel 18

(S)-2,2-Dimethyl-1,3-dioxolan-4-methanol aus 5,6-O-Isopropyliden-D-isoascorbinsäure

Analog zu Beispiel 12 wurde aus 5,6-O-Isopropyliden-D-isoascorbinsäure durch Oxidation mit Wasserstoffperoxid in Gegenwart von Calciumcarbonat und Natriumcarbonat und anschliessende Elektrolyse das (S)-2,2-Dimethyl-1,3-dioxolan-4-methanol hergestellt.
Ausbeute: 58,2%
Siedepunkt: 75 bis 78 ° C/12 Torr
$[\alpha]_D^{20}$ = +11,5 ° (c = 5, Methanol);
$[\alpha]_D^{20}$ = +15,1 ° (ohne Lösungsmittel)
$^1$H-NMR: ($CDCl_3$, 300 MHz) δ
1,39 (s, 3H); 1,46 (s, 3H); 2,32 (s, 1H, OH); 3,61 (dd, J = 11,5/5 Hz, 1H, H-C(3)); 3,74 (dd, J = 11,5/4 Hz, 1H, H-C(3)); 3,80 (dd, J = 8/6,5 Hz, 1H, H-C(1)); 4,04 (dd, J = 8/6,5 Hz, 1H, H-C(1)); 4,25 (tdd, J = 6,5/5/4 Hz, 1H, H-C(2)).

Beispiel 19

(R)-4-Isopropylaminomethyl-2,2-dimethyl-1,3-dioxolan aus 5,6-O-Isopropyliden-L-ascorbinsäure

Eine nach Beispiel 12 hergestellte Lösung von (S)-2,2-Dimethyl-1,3-dioxolan-4-carbaldehyd (Mengen und Durchführung wie in Beispiel 12, jedoch ohne Reduktion mit Natriumborhydrid) wurde innerhalb von 3 Stunden unter Wasserstoffatmosphäre zu einem Gemisch von 20 ml Isopropylamin, 2,0 g Palladium/Aktivkohle (10% Pd) und 200 ml Methanol getropft. Das Gemisch wurde in einer Schüttelapparatur bei 3 bar Wasserstoffdruck und Raumtemperatur bis zur Beendigung der Wasserstoffaufnahme hydriert. Anschliessend wurde der Katalysator abfiltriert und das Filtrat auf 100 ml eingeengt. Nach Zugabe von 10 g Natriumcarbonat wurde das Gemisch fünfmal mit je 50 ml Dichlormethan extrahiert, die Dichlormethanphasen über Natriumsulfat getrocknet und das Lösungsmittel abdestilliert. Der ölige Rückstand wurde anschliessend im Vakuum destilliert.
Ausbeute: 6,8 g (41,4%, bezogen auf 5,6-O-Isopropyliden-L-ascorbinsäure)
Siedepunkt: 43 bis 45 ° C/0,1 Torr
$[\alpha]_D^{20}$ = +7,3 ° (c = 2, Methanol)

## Patentansprüche

1. Verfahren zur Herstellung von enantiomerenreinen 2,2,4-trisubstituierten 1,3-Dioxolanen der allgemeinen Formel

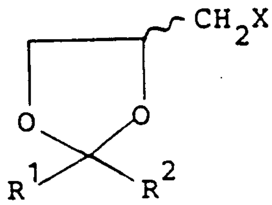

I

in welcher $R^1$ und $R^2$ entweder gleich und

a) Wasserstoff oder
b) Alkylgruppen mit 1 bis 4 C-Atomen oder
c) Arylgruppen oder
d) Arylalkylgruppen

oder $R^1$ und $R^2$ zusammen eine 1,4-Butandiyl- oder 1,5-Pentandiylgruppe und X entweder eine Hydroxygruppe oder, unter der Voraussetzung, dass $R^1$ und $R^2$ keine Arylgruppen sind, $NHR^3$ mit $R^3$ = Alkyl mit 1 bis 8 C-Atomen oder Aryl sind, dadurch gekennzeichnet, dass man je nach gewünschter Konfiguration eine entsprechend substituierte Threonsäure oder Erythronsäure der allgemeinen Formel

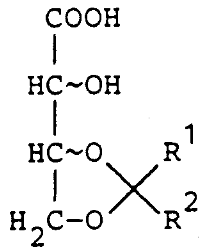

II

oder ein Salz davon durch Elektrolyse in den entsprechend substituierten 1,3-Dioxolan-4-carbaldehyd der allgemeinen Formel

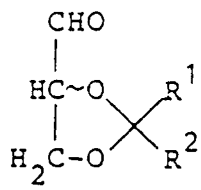

III

überführt und diesen, ohne ihn zu isolieren, durch Reduktion oder reduktive Aminierung in das enantiomerenreine 2,2,4-trisubstituierte 1,3-Dioxolan gemäss Formel I überführt.

**2.** Verfahren nach Patentanspruch 1, dadurch gekennzeichnet, dass die Elektrolyse in wässriger Lösung bei pH 4 bis 10 durchgeführt wird.

**3.** Verfahren nach einem oder beiden der Patentansprüche 1 und 2, dadurch gekennzeichnet, dass die Elektrolyse in Gegenwart eines tertiären Amins durchgeführt wird.

**4.** Verfahren nach einem oder beiden der Patentansprüche 1 und 2, dadurch gekennzeichnet, dass die Threon- oder Erythronsaure in Form eines Alkali- oder Erdalkali-Salzes eingesetzt wird.

**5.** Verfahren nach einem oder mehreren der Patentansprüche 1 bis 4, dadurch gekennzeichnet, dass die Threon- oder Erythronsäure durch oxidativen Abbau einer entsprechend substituierten Ascorbinsäure oder Isoascorbinsäure der allgemeinen Formel:

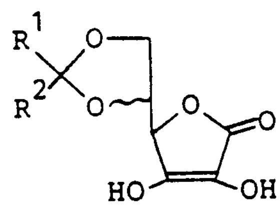

IV

mit wässrigem Wasserstoffperoxid in Gegenwart von Calciumcarbonat hergestellt wird.

**6.** Verfahren nach Patentanspruch 5, dadurch gekennzeichnet, dass die Oxidation der Ascorbinsäure oder Isoascorbinsäure in Gegenwart eines Alkalicarbonats erfolgt.

## Claims

**1.** A process for the preparation of pure enantiomers of 2,2,4-trisubstituted 1,3-dioxolanes of the general formula

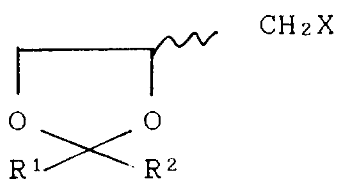

I

wherein either $R^1$ and $R^2$ are the same and

a. hydrogen or
b. alkyl groups with 1 to 4 C atoms or
c. aryl groups or
d. aryl alkyl groups

or $R^1$ and $R^2$ together are a 1,4-butane diyl group or a 1,5-pentane diyl group and X is either a hydroxy group or - on condition that $R^1$ and $R^2$ are not aryl groups - $NHR^3$ with $R^3$ = alkyl with 1 to 8 C-atoms or aryl,

characterized in that depending on the desired configuration a correspondingly substituted threon acid or erythron acid of the general formula

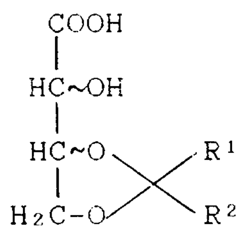

II

or a salt thereof are converted through electrolysis into the correspondingly substituted 1,3-dioxolane-4-carbaldehyde of the general formula

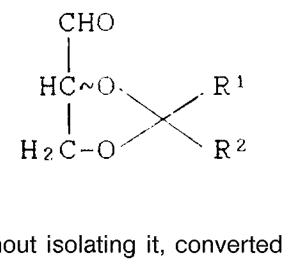

III

which is then, without isolating it, converted through reduction or reductive amination into the pure

enantiomer of 2,2,4-trisubstituted 1,3-dioxolane according to formula I.

2. A process as in claim 1, characterized in that the electrolysis is carried out in aqueous solution at pH 4 to 10.

3. A process as in one or both of the claims 1 and 2, characterized in that the electrolysis is carried out in the presence of a tertiary amine.

4. A process as in one or both of the claims 1 and 2, characterized in that the threon acid or erythron acid are employed in the form of an alkali salt or an alkaline earth salt.

5. A process as in one or more of the claims 1 to 4, characterized in that the threon acid or erythron acid is prepared through oxidative degradation of a correspondingly substituted ascorbic acid or isoascorbic acid of the general formula:

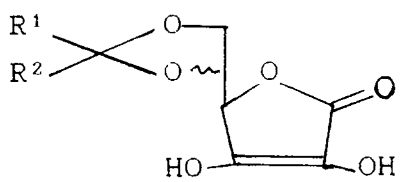

IV

with aqueous hydrogen peroxide in the presence of calcium carbonate.

6. A process as in claim 5, characterized in that the oxydation of the ascorbic acid or isoascorbic acid is carried out in the presence of an alkali carbonate.

**Revendications**

1. Procédé pour la préparation d'énantiomères purs de 1,3-dioxolanes, 2,2,4-trisubstitués exempts d'énantiomères de la formule générale

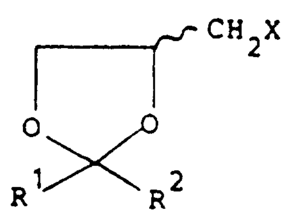

I

dans laquelle $R^1$ et $R^2$ sont ou bien identiques et sont
a) l'hydrogène ou sont
b) des groupes alkyle avec 1 à 4 atomes C ou
c) des groupes aryle ou
d) des groupes arylalkyle

ou $R^1$ et $R^2$ sont conjointement un groupe 1,4-butandiyle ou 1,5-pentadiyle et X est ou bien un groupe hydroxy ou, à condition que $R^1$ et $R^2$ ne soient pas des groupes aryle, $NHR^3$ avec $R^3$ = alkyle avec 1 jusqu'à 8 atomes de carbone ou sont aryle, caractérisé en ce que selon la configuration souhaitée, on transforme un acide thréonique substitué de façon correspondante ou un acide érythronique de formule générale

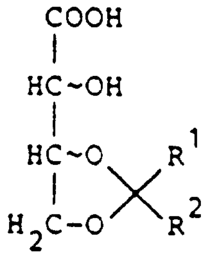

II

ou son sel par électrolyse en le 1,3-dioxalane-4-carbaldéhyde substitué de la formule générale

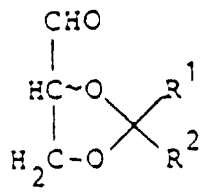

III

et l'on transforme celui-ci, sans l'isoler, par réaction ou amination réductrice en le 1,3-dioxalane, 2,2,4-trisubstitué exempt d'énantionères selon la formule I.

**2.** Procédé selon la revendication 1, caractérisé en ce que l'électrolyse est effectuée en solution aqueuse à un pH de 4 à 10.

**3.** Procédé selon une ou deux des revendications 1 et 2, caractérisé en ce que l'électrolyse est effectuée en présence d'une amine tertiaire.

**4.** Procédé selon une ou deux des revendications 1 et 2, caractérisé en ce que les acides thréoniques ou érythroniques sont mis en oeuvre sous forme d'un sel alcalin ou alcalino-terreux.

**5.** Procédé selon une ou plusieurs des revendications 1 à 4, caractérisé en ce que l'acide thréonique ou érythronique est préparé par réduction oxydatrice d'un acide ascorbinique ou isoascorbinique substitué de façon correspondante de la formule générale

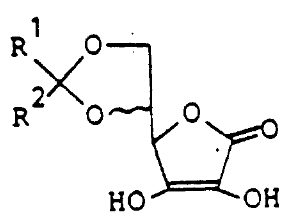

IV

avec du peroxyde d'hydrogène aqueux en présence de carbonate de calcium.

**6.** Procédé selon la revendication 5, caractérisé en ce que l'oxydation de l'acide ascorbinique ou de l'acide isoascorbinique s'effectue en présence d'un carbonate alcalin.